# EUROPEAN PATENT SPECIFICATION

(11) **EP 2 898 834 B1**
(45) Date of publication and mention of the grant of the patent: **07.11.2018**
(21) Application number: 12884846.2
(22) Date of filing: 18.09.2012
(51) Int. Cl.: A61B 10/00, G01M 7/02, A61B 5/00

(54) **DEVICE FOR EVALUATING DIABETIC PERIPHERAL NEUROPATHY**
VORRICHTUNG ZUR BEURTEILUNG VON DIABETISCHER PERIPHERER NEUROPATHIE
DISPOSITIF POUR ÉVALUER LA NEUROPATHIE PÉRIPHÉRIQUE DIABÉTIQUE

(43) Date of publication of application: 29.07.2015
(73) Proprietor: National Institute of Advanced Industrial Science and Technology, Tokyo 100-8921 (JP); Showa University, Tokyo 142-8555 (JP); Asuka Electric Co. Ltd., Osaka-shi, Osaka 5310041 (JP); Takahashi, Noriyo, Amagasaki-shi, Hyogo 6610012 (JP)
(72) Inventor: INO, Shuichi, Tsukuba-shi Ibaraki 305-8566 (JP); SATO, Mitsuru, Tokyo 142-8555 (JP); TAKAHASHI, Noriyo, Amagasaki-shi, Hyogo 6610012 (JP); YOSHIMURA, Shinichi, Osaka-shi Osaka 531-0041 (JP)
(74) Representative: Plougmann Vingtoft a/s
(86) International application number: PCT/JP2012/073835
(87) International publication number: WO 2014/045339

(56) References cited:
- WO-A1-97/06730
- JP-A- 2007 512 900
- JP-A- 2008 517 648
- JP-A- 2009 529 963
- JP-A- 2010 011 984
- JP-A- 2010 022 585
- JP-B1- 4 611 453
- US-A1- 2006 004 302
- US-A1- 2006 178 596
- US-A1- 2012 220 892

## Description

### TECHNICAL FIELD

The present invention relates to an evaluation apparatus for quantitatively evaluating peripheral neuropathy originating in type 2 diabetes (hereinafter, simply referred to as diabetes), in particular, sensory disturbance in a sole. A quantitative evaluation result obtained by the present invention can be used as an index for prediction of diabetes of early stages.

### BACKGROUND ART

Diabetes occupying a major proportion of lifestyle-related diseases is difficult to be found in its early stages and to be treated appropriately because it exhibits no obvious symptoms in its early stages. For medically identifying diabetes, generally, a blood test is conducted to examine whether or not a blood sugar level exceeds a normal level. However, patients suffering from early-stage diabetes who have no outstanding symptoms and lack differences from healthy persons in terms of appearance do not feel the need of undergoing a blood test of their own accords, and hence diabetes is rarely found in its early stages. Many patients suffering from diabetes with such weak symptoms would remain unfound, and a survey conducted by a public organization in 2007 reports that people who are highly suspicious of diabetes and people whose possibility of diabetes cannot be denied count up to 22.1 million in Japan.

Diabetes is known to be associated with complications such as neuropathy (peripheral neuropathy), retinopathy and nephropathy depending on the degree of progression. Among these complications, neuropathy is known to appear from distal portions of extremities in early stages of diabetes in such a fashion that the sensation to an external stimulus to a sole that has been felt before gradually reduces, and the sensation is finally lost. However, since the reduction in sensation to an external stimulus of a constant intensity is modest, the reduction in sensation in a sole is not clearly recognized in everyday life as a subjective symptom, and in quite a number of cases, a diagnosis of diabetes is made only after severe neuropathy, or severer complications such as retinopathy and nephropathy are developed.

The present invention can suggest the possibility of diabetes for diabetes in early stages associated with no subjective symptoms by quantitatively measuring sensory disturbance in a sole and making an evaluation. As such an evaluation method for skin sensation, a Semmes-Weinstein skin sensation meter is well known in the art. The skin sensation meter having a probe head quantifies tactile sensation and pain sensation by attaching 20 kinds of filaments having different thicknesses in turn to the probe head, and identifying a threshold according to which filament caused tactile sensation or pain sensation when it is pushed against the tip of a finger. Applications of the skin sensation meter include, for example, evaluation of degree of postoperative recovery of nerves in the field of orthopedics.

Regarding the present invention, an apparatus for measuring and quantifying human skin sensation is known from, for example, Patent Documents 1 to 3. A vibration sensory threshold measuring device in Patent Document 1 includes a pedestal for supporting the forearm of a subject, a vibration exciter for applying a vibration stimulus to the tip of a finger of a subject, a push-button switch to be operated by a subject, and a controller for calculating a threshold based on output signals from a lord cell of the vibration exciter and an acceleration meter to make evaluation. The vibration sensory threshold measuring device in Patent Document 1 is based on the measurement method defined by JIS-8-7763, and is featured in that the magnitude of vibration stimulus applied to a subject is varied randomly within a predetermined range with respect to a top-down method threshold determined by the last top down method.

Patent Document 2 discloses a load measuring apparatus for measuring a human skin sensory threshold. The apparatus includes a pedestal for supporting a part to be measured of a subject, a strut fixed to the pedestal, a movable table capable of moving vertically along the strut, a micro load converter fixed to the movable table, a measuring needle provided in the micro load converter, and a controller. The controller controls drive condition of a stepping motor for driving the movable table up and down, and processes a signal output from the micro load converter.

Patent Document 3 discloses an apparatus for testing recognition of human skin sensation, likewise the load measuring apparatus of Patent Document 2. This testing apparatus includes a pair of right and left slide blocks, a deflection block fixed to the front end of each of the blocks, a probe fixed to the front end of each of the deflection blocks, a structure for adjusting the left-right distance of the slide blocks, and a strain gauge for detecting strain of the deflection block. For testing recognition of skin sensation, the left-right distance of the pair of probes is set to a predetermined condition, and a tip end of the probe is dragged while it is pushed against the skin with predetermined power, and whether or not the subject recognizes sensation is examined.

Patent Document 4 discloses a test device for testing a so-called diabetic foot that exhibits little or no sensation of a sole due to concurrence of neuropathy. This test device includes a box-shaped housing, a transparent foot platen disposed on the top face of the housing, a video camera and a light source disposed inside the housing, and a transmitter for transmitting audio data of the video camera. For conducting a test, in the condition that a sole of a subject is placed at a predetermined position of the foot platen, an image of the sole is captured with a video camera, and the transmitted image is analyzed by a physician, and thus the degree of neuropathy is diagnosed.

US 2012/0220892 describes a vibrometer capable of applying vibrotactile stimulation to a test subject's skin to determine the existence of peripheral neuropathy.

### PRIOR ART DOCUMENTS

### PATENT DOCUMENTS

Patent Document 1: JP 4611453 B1 (paragraphs 0025 to 0027, Fig. 1)
Patent Document 2: JP 06-30904 A (paragraph 0016, Fig. 1)
Patent Document 3: JP 05-503022 W (p.3, left lower column, lines 18 to 20, Fig. 2)

### DISCLOSURE OF THE INVENTION

### PROBLEMS TO BE SOLVED BY THE INVENTION

Although measuring apparatuses as disclosed in Patent Documents 1 to 3 are known as apparatuses for measuring a threshold of human skin sensation, any of these merely measures the degree of a sensory threshold when an external stimulus is applied to a subject's skin. Therefore, while these measurement results can be used for evaluating, for example, a sensory threshold when a peripheral nerve is injured, they are difficult to be applied in other ways. Further, in using the sensory threshold measuring apparatus of Patent Document 1, since the measurement condition is strictly defined by the JIS standard, only a physician or a specialized technical expert is permitted to make measurement. For example, the position of the probe to be brought into contact with the tip of a finger should not be the part where the skin is thick, and should be located in the part excluding the center position of the finger print. Also, the measurement condition is very complicated as represented by the fact that the depth of a recess in skin in a static state when the probe is brought into abutment with the tip of the finger should be 1.5 ± 0.8 mm.

In the load measuring apparatus of Patent Document 2, the measurement needle is pushed against the skin by lowering the movable table at a constant speed by means of the stepping motor, and the load of the measurement needle when the subject senses the stimulus by the measurement needle is measured by a load measuring part of the micro load converter, and this load is taken as a sensory threshold. Therefore, for identifying a sensory threshold of a sole, for example, it is necessary to repeatedly apply a stimulus with the measurement needle at a large number of measurement points, and plenty of time is required to identify the presence or absence, and the degree of peripheral neuropathy.

Regarding the sensation recognizing apparatus of Patent Document 3, for example, a physician pushes the tip end of the probe against the skin with predetermined power while holding the entire case with one hand, and examines whether or not the subject recognizes sensation by dragging the probe in the above condition. Therefore, it is difficult to make the pushing power against the skin and the dragging speed of the probe constant, and noise is likely to be involved during dragging of the probe. Therefore, this apparatus is suited for general determination of a sensory threshold, but not for accurate measurement of a sensory threshold.

In the test device for testing a diabetic foot disclosed in Patent Document 4, a physician analyzes an image transmitted from the test device to diagnose the degree of neuropathy. This test device merely examines the presence or absence of a wound in the sole for patients having little or no sensation in the sole. Therefore, it is impossible to identify the presence or absence of neuropathy, or the degree of progression of neuropathy and to determine the possibility of diabetes in early stages of diabetes accompanying no subjective symptoms.

A medical examination for diabetes of initial stage is mainly conducted as an outpatient examination. And regarding quantitative evaluation of neuropathy (sensory disturbance) of a sole in examination scenes, simple evaluation (test) technique, a short evaluation time, as well as accurate examination by anyone without the need of specialized knowledge or technique are demanded. These conventional measuring apparatuses still have a room for improvement in the point that not everyone can easily measure a sensory threshold because the measurement condition is complicated and a long time is required for measurement, and only a physician or a technician having a specialized knowledge is allowed to use these measuring apparatuses.

In quantitatively evaluating neuropathy in a sole by stimulating skin sensation of the sole, how the skin sensation is stimulated is a determinative factor of the evaluation. In this type of sensitivity test, as can be seen in Patent Document 1, there is known a bottom-up method in which a stimulus is repeatedly applied to a subject while the stimulus intensity is gradually increased from an imperceptible micro stimulus, and a threshold of skin sensation is identified according to the point at which the stimulus is recognizable, and a top down method in which the stimulus intensity is gradually decreased, and a method of limits using both of these. Also known is a constant stimulus method in which stimulus intensity is changed randomly. While the method of limits can advantageously measure a threshold with a smaller number of stimulus applications, it is likely to involve measurement errors due to habituation and expectation. In this respect, the constant stimulation method in which stimulus intensity is changed randomly can eliminate the influences of habituation and expectation, however, it is not suited for evaluating sensory disturbance in a short examination time because it requires a long time for measurement. Thus, it is inevitable to employ the method of limits for realizing the outpatient examination.

For appropriately evaluating neuropathy in a sole by stimulating skin sensation of the sole, it is impossible to obtain appropriate evaluation by applying to a subject a stimulus of the same condition as that applied to a healthy person. This is because for clearly grasping diabetic neuropathy, the range of intensities of presented sensory stimuli for the subject is much wider than that for the healthy person. For example, when stimulus intensity of sensory stimulus for a subject is defined by a moving distance of a probe, the test can be carried out by moving the probe within the range from 5 µm to 100 µm for a healthy person. However, a clinical test conducted by the present inventor revealed that stimulus intensity exceeding the range of 5 µm to 1600 µm need to be applied to a subject with neuropathy originating in diabetes. This means that the variation range of stimulus intensity should be set much wider than that for a healthy person in order to equally evaluate the degree of neuropathy originating in diabetes. For this reason, a long time is required for appropriately evaluating the degree of neuropathy even with the method of limits as described above, and this method is not suited for the outpatient examination.

As a result of diligent effort based on the aforementioned findings, inventors of the present invention found that by determining a variation range of stimulus intensity by using known reference data of sensory thresholds obtained by applying a moving stimulus to a sole of patients suffering from diabetes, and an age correction factor calculated from a standard value of sensory thresholds based on the different ages of patients, it is possible to determine a sensory threshold with a smaller number of stimulus applications, and further it is possible to identify the presence or absence of neuropathy or the status of progression of neuropathy from the obtained sensory threshold, and proposed the present invention.

It is an object of the present invention to provide an evaluation apparatus for diabetic peripheral neuropathy capable of quantitatively measuring a sensory threshold in a sole resulting from diabetes, and automatically identifying the presence or absence of neuropathy or the status of progression of neuropathy according to the measurement result.

It is another object of the present invention to provide an evaluation apparatus for diabetic peripheral neuropathy suited for an outpatient examination capable of automatically identifying the presence or absence of neuropathy or the status of progression of neuropathy with a smaller number of stimulus applications, and thus requiring a shorter time for evaluation, and allowing anyone to accurately conduct the test without the need of specialized knowledge or technique.

### SOLUTION TO THE PROBLEMS

The objects of the present invention are achieved with an evaluation apparatus according to the independent claim. An evaluation apparatus for diabetic peripheral neuropathy according to the present invention includes a measuring device A for measuring a sensory threshold of a sole, and a main controller B for identifying a sensory threshold from a measurement result of the measuring device A and evaluating presence or absence of neuropathy or status of progression of neuropathy from the identified sensory threshold. The measuring device A includes a foot pedestal 2 disposed on a base 1, for supporting a sole of a subject who is sitting or standing, a probe 4 for applying a moving stimulus to the sole, a probe driving structure 3 for operating movement of the probe 4, an input switch 5 to be operated by the subject recognizing the moving stimulus, and a drive controller 51 for controlling drive condition of the probe driving structure 3. The main controller B preliminarily stores reference data of known sensory thresholds obtained by applying a moving stimulus to a sole of patients, and age correction factors calculated from standard values of sensory thresholds based on different ages of patients. The drive controller 51 controls drive condition of the probe driving structure 3 by using the reference data and the age correction factors to sequentially conduct a primary stimulus applying condition, a secondary stimulus applying condition, and a tertiary stimulus applying condition, thereby measuring a sensory threshold. In the primary stimulus applying condition, a rough sensory threshold is temporarily set by setting a variation range of moving stimulus by the probe 4 large. In the secondary stimulus applying condition, taking the temporarily set rough sensory threshold as a reference value, a moving stimulus having a small variation range is applied to measure a sensory threshold. In the tertiary stimulus applying condition, when a stimulus response by the subject is not observed in the secondary stimulus applying condition, a moving stimulus larger than the moving stimulus corresponding to the sensory threshold temporarily set in the primary stimulus applying condition is applied and a sensory threshold is measured. The main controller B compares and evaluates the measured sensory threshold and the aforementioned known reference data to automatically determine the presence or absence of neuropathy in the sole and the degree of neuropathy.

The probe driving structure 3 includes a first table 11 and a second table 12 that are guided and supported while they are reciprocally slidable in a front-back direction and in a left-right direction, a first driving mechanism 13 disposed on the base 1, for reciprocally operating the first table 11, a second driving mechanism 14 disposed on the first table 11, for reciprocally operating the second table 12, and a probe fixing portion 40 disposed on the second table 12. The probe 4 attached to the probe fixing portion 40 is separately moved in a moving direction of the first table 11 and in a moving direction of the second table 12 to measure a moving distance and a moving speed of the probe 4 as independent variables. The measured sensory threshold and the reference data are evaluated by the main controller B.

The probe driving structure 3 includes a moving table 11 that is guided and supported while it is reciprocally slidable, a driving structure 13 disposed on the base 1, for reciprocally operating the moving table 11, and a probe fixing portion 40 disposed on the moving table 11. Posture of the subject is changed between the posture along the moving direction of the probe 4 attached to the probe fixing portion 40 and the posture perpendicular to the moving direction of the probe 4, and thus an front-back moving stimulus and a left-right moving stimulus are applied to a sole.

The evaluation apparatus includes display means 55 that displays a determination result of the main controller B regarding the presence or absence of neuropathy in a sole, and the degree of neuropathy.

A motor 23 constituting the first driving mechanism 13 and a motor 33 constituting the second driving mechanism 14 are respectively fixed to brackets 27, 37 via vibration insulating structures 28, 38 that block vibration. Rotary power of the motors 23, 33 is converted into reciprocating operation by means of ball screw shafts 21, 31, and male screw bodies 22, 32 fixed to the first table 11 and the second table 12 to reciprocally slide the first table 11 and the second table 12 in the front-back direction and the left-right direction.

An evaluation method for diabetic peripheral neuropathy includes a test preparing step of placing a sole on a contact window 8 opening at a predetermined position of a foot pedestal 2, a stimulating and measuring step of operating a probe 4 to move by a probe driving structure 3 that operates according to a control procedure of a drive controller 51 and measuring a sensory threshold by operating the input switch 5 when the subject feels a moving stimulus in the sole, and an evaluating step of evaluating the measured sensory threshold by a main controller B. The main controller B preliminarily stores reference data of known sensory thresholds obtained by applying a moving stimulus to a sole of patients, and age correction factors calculated from standard values of sensory thresholds based on different ages of patients. The drive controller 51 in the stimulating and measuring step controls drive condition of the probe driving structure 3 by using the reference data and the age correction factors to conduct measurement in the primary stimulus applying condition where a rough sensory threshold is temporarily set by setting the variation range of moving stimulus by the probe 4 large. Then taking the rough sensory threshold obtained in the primary stimulus applying condition as a reference value, it conducts measurement in the secondary stimulus applying condition where a moving stimulus having a small variation range is applied to measure a sensory threshold. When a stimulus response by the subject is not observed in the secondary stimulus applying condition, it conducts measurement in the tertiary stimulus applying condition where a moving stimulus larger than the moving stimulus corresponding to the sensory threshold temporarily set in the primary stimulus applying condition is applied and a sensory threshold is measured. In other words, the primary stimulus applying condition, the secondary stimulus applying condition, and the tertiary stimulus applying condition are conducted in this order. In the evaluating step, the main controller B compares and evaluates the measured sensory threshold and the aforementioned known reference data to automatically determine the presence or absence of neuropathy in the sole and the degree of neuropathy.

In the stimulating and measuring step, when a stimulus response of the subject is not observed in the primary stimulus applying condition, the condition shifts to a quaternary stimulus applying condition where a moving stimulus of the maximum intensity is applied to measure a sensory threshold. When a stimulus response of the subject is observed in the quaternary stimulus applying condition, the tertiary stimulus applying condition where a moving stimulus having a small variation range is applied to measure a sensory threshold while taking the maximum moving stimulus as a reference value is conducted.

In the stimulating and measuring step, the probe 4 is separately moved in the front-back direction and in the left-right direction by the probe driving structure 3 to apply an front-back moving stimulus and a left-right moving stimulus to the sole, and thus a moving distance and a moving speed of the probe 4 are measured as independent variables.

In the stimulating and measuring step, a moving stimulus is separately applied to each of a hallux face E1, a thenar face E2, and a heel face E3 of a sole to measure a sensory threshold.

### ADVANTAGEOUS EFFECT OF THE INVENTION

In the evaluation apparatus of the present invention, the probe 4 is moved by operation of the probe driving structure 3 to apply a moving stimulus to the sole of the subject, and a sensory threshold for the moving stimulus is measured on the basis of an output signal of the input switch 5 operated by the subject. Also in the step of measuring a sensory threshold, the drive condition of the probe driving structure 3 is controlled by using preliminarily stored known reference data of sensory thresholds and age correction factors, and a sensory threshold is measured through the primary to tertiary stimulus applying conditions, and the obtained sensory threshold and the reference data are compared and evaluated by the main controller B to automatically determine the presence or absence of neuropathy in a sole, and the degree of neuropathy.

As described above, according to the evaluation apparatus of the present invention, only by placing a sole of a subject at a predetermined position of the foot pedestal 2, and actuating the evaluation apparatus after inputting age data, it is possible to make the probe 4 be automatically moved by the probe driving structure 3, and to apply a moving stimulus as set for the subject accurately by the set procedure. Therefore, it is possible to measure a sensory threshold with high reproducibility by eliminating any variation in moving stimulus for the subject, and to quantitatively measure a sensory threshold in a sole resulting from diabetes, and to automatically identify the presence or absence of neuropathy, or the status of progression of neuropathy according to the measurement result. Since a sequence of measurement and evaluation is conducted automatically, a sensory threshold can be measured easily even by a measurer who does not have specialized knowledge of medicine or specialized knowledge and technique of bio-medical instrumentation for measuring and evaluating a sensory threshold in a sole, and additionally, variation in the measurement result between different measurers can be eliminated. Therefore, it is possible to provide an evaluation apparatus for diabetic peripheral neuropathy especially suited for an outpatient examination for which a sufficient time is difficult to be taken.

After temporarily setting a rough sensory threshold in the primary stimulus applying condition, a sensory threshold is measured more specifically by applying a moving stimulus (secondary stimulus) having a small variation range while taking the temporarily set rough sensory threshold as a reference value. Therefore, it is possible to determine a sensory threshold accurately with a less number of stimulus applications. This makes it possible to measure and evaluate a sensory threshold in a short time by largely reducing the time required for evaluation of a sensory threshold in outpatient examination for which a sufficient time is difficult to be taken. Note that the tertiary stimulus application is conducted for the purpose of measuring a sensory threshold again by applying a large moving stimulus because when a stimulus response of the subject is not observed in the secondary stimulus applying condition, some errors or mistakes can be involved in measurement of the rough sensory threshold temporarily set in the primary stimulus applying condition.

By making up the probe driving structure 3 from the first table 11 and the second table 12, and the first and the second driving structures 13, 14 for driving these tables 11, 12 and so on, it is possible to apply an front-back moving stimulus and a left-right moving stimulus to a sole appropriately. In this manner, by measuring the presence or absence of perception of the subject with respect to the moving stimuli of the two directions by separately moving the probe 4 in the two directions, it is possible to measure a sensory threshold for a moving stimulus in a shearing direction applied to the sole in contrast to the case of a conventional measuring device that applies an oscillation stimulus or a compression stimulus on a specific site of skin. Further, it is possible to obtain a measurement result reflecting the finding that the perception characteristics of a sole differ between the two directions, and also it is possible to measure a moving distance and a moving speed of the probe 4 as independent variables. Therefore, by comparing the obtained measurement result with known sensory thresholds that are preliminarily collected and put onto a database, it is possible to identify the presence or absence of neuropathy originating in diabetes, or the status of progression of neuropathy more accurately.

According to the probe driving structure 3 that drives only one moving table 11, it is possible to make the entire structure of the evaluation apparatus very simple and compact, and thus it is possible to provide an evaluation apparatus suited for use in a narrow space such as a consultation room or a waiting room. Simplification of the structure of the evaluation apparatus is also advantageous in that the total cost can be reduced, and the cost for installation of the evaluation apparatus can be reduced. Further, since it is possible to apply an front-back moving stimulus and a left-right moving stimulus to a sole only by changing the posture of the subject between the posture along the moving direction of the probe 4 and the posture perpendicular to the moving direction of the probe 4, it is possible to obtain a measurement result reflecting the finding that the perception characteristics of a sole differ between the two directions as is the case with the form of the probe driving structure 3 that drives the two tables 11, 12. Therefore, by comparing the obtained measurement result with known sensory thresholds that are preliminarily collected and put onto a database, it is possible to identify the presence or absence of neuropathy originating in diabetes, or the status of progression of neuropathy more accurately.

The display means 55 provided in the evaluation apparatus makes it possible to concretely explain the presence or absence of neuropathy originating in diabetes, or the status of progression of neuropathy to the subject while presenting a determination result compared and evaluated by the main controller B on the display means 55. For example, in the case where a determination result is rated on 10-point scale, the evaluation point among the 10 points is indicated for an outpatient clinician, and also an evaluation result depending on the evaluated point, for example, "Slight neuropathy is observed", or "Strongish neuropathy is observed" can be displayed and presented for the subject.

According to the measuring device that activates the motors 23, 33 of the first driving mechanism 13 and the second driving mechanism 14 by an operation of a start button of the main controller B to automatically apply a series of moving stimuli, it is possible to drive the probe 4 according to a predetermined procedure and apply a moving stimulus accurately as preliminarily set for the subject only by turning on the start button. Incidentally, measurement of a sensory threshold is a troublesome task because stimuli of various intensities should be applied to a single site many times, and these operations should be repeated for other site. Therefore, it is difficult to conduct measurement in clinical practice where the time is limited. However, by automating application of a moving stimulus to a subject, and recording of a sensory threshold of the subject by controlling operations of both of the driving structures 13, 14 by the drive controller 51 as described above, a measurer not having specialized knowledge and technique can easily measure a sensory threshold, and variation in measurement result between different measurers can be eliminated, and thus an evaluation apparatus for diabetic peripheral neuropathy suited for an outpatient examination can be provided.

In the evaluation method measurement of a sensory threshold of a sole and evaluation of the measurement result are conducted through a test preparing step, a stimulating and measuring step, and an evaluating step. In the stimulating and measuring step, the drive condition of the probe driving structure 3 is controlled by using preliminarily stored reference data of known sensory thresholds and age correction factors, and a sensory threshold can be measured and determined through the primary to tertiary stimulation applying conditions. Also in the evaluating step, it is possible to automatically determine the presence or absence of neuropathy of a sole, and the degree of neuropathy by comparing and evaluating the sensory threshold obtained in the previous step, and the reference data in the main controller B.

As described above, according to the evaluation method, by placing a sole of a subject at a predetermined position of the foot pedestal 2, and starting the stimulating and measuring step after inputting age data, it is possible to make the probe 4 be automatically moved by the probe driving structure 3, and to apply a moving stimulus as set for the subject accurately by the set procedure. Therefore, it is possible to measure a sensory threshold with high reproducibility by eliminating any variation in moving stimulus for the subject, and to quantitatively measure a sensory threshold in a sole resulting from diabetes, and to automatically identify the presence or absence of neuropathy, or the status of progression of neuropathy according to the measurement result. Further, since the sequence of measurement and evaluation is conducted automatically, a sensory threshold can be measured easily by a measurer who does not have specialized knowledge of medicine or specialized knowledge and technique of bio-medical instrumentation for measuring and evaluating a sensory threshold in a sole, and additionally, variation in the measurement result between different measurers can be eliminated. Therefore, it is possible to provide an evaluation method for diabetic peripheral neuropathy especially suited for an outpatient examination for which a sufficient time is difficult to be taken.

Further, after temporarily setting a rough sensory threshold in the primary stimulus applying condition, a sensory threshold is measured more specifically by applying a moving stimulus (secondary stimulus) having a small variation range while taking the temporarily set rough sensory threshold as a reference value. Therefore, it is possible to determine a sensory threshold accurately with a less number of stimulus applications. This makes it possible to measure and evaluate a sensory threshold in a short time by largely reducing the time required for evaluation of the sensory threshold in an outpatient examination for which a sufficient time is difficult to be taken. Note that the tertiary stimulus application is conducted for the purpose of measuring a sensory threshold again by applying a large moving stimulus because when a stimulus response of the subject is not observed in the secondary stimulus applying condition, some errors or mistakes can be involved in measurement of the rough sensory threshold temporarily set in the primary stimulus applying condition.

In the stimulating and measuring step, when a stimulus response of the subject is not observed in the primary stimulus applying condition, the condition shifts to the quaternary stimulus applying condition where a sensory threshold is measured by applying a moving stimulus of the maximum intensity for the purpose of checking whether or not a stimulus response of the subject to the moving stimulus of the maximum intensity is observed. This also serves to securely measure and identify the sensory threshold by shifting the condition to the tertiary stimulus applying condition where a sensory threshold is measured by applying a moving stimulus having a small variation range while taking the maximum moving stimulus as a reference value, when a stimulus response of the subject to the moving stimulus of the maximum intensity is observed. By providing the quaternary stimulus applying condition in this manner, it is possible to distinctly evaluate neuropathy associated with especially high degree of sensory disturbance by identifying the state that the subject does not show a stimulus response to the moving stimulus of the maximum intensity, namely having anesthesia, and the state that the subject has very severe sensory disturbance.

In the stimulating and measuring step, by applying a front-back moving stimulus and a left-right moving stimulus to a sole, and measuring a moving distance and a moving speed of the probe 4 as independent variables, it is possible to measure a sensory threshold for a moving stimulus in the shearing direction applied to the sole in contrast to the case of a conventional measuring device that applies an oscillation stimulus or a compression stimulus on a specific site of skin. Further, it is possible to obtain a measurement result reflecting the finding that the perception characteristics of a sole differ between the two directions, and also it is possible to measure a moving distance and a moving speed of the probe 4 as independent variables. Therefore, by comparing the obtained measurement result with known sensory thresholds that are preliminarily collected and put onto a database, it is possible to evaluate the presence or absence of neuropathy originating in diabetes, or the status of progression of neuropathy more accurately.

Sensory thresholds of the hallux face E1, the thenar face E2, and the heel face E3 of a sole are measured in the stimulating and measuring step because sensory receptors in the skin that sense mechanical moving stimuli are densely distributed in these measurement target sites, and thresholds to sensory stimuli are different among these sites. Also, sensory thresholds of these sites can be measured more accurately compared with the case where other site is set as a measurement target site, and thus the presence or absence of neuropathy, or the status of progression of the neuropathy can be evaluated more easily.

### BRIEF DESCRIPTION OF THE DRAWINGS

Fig. 1 is a longitudinal lateral view showing a sensory threshold measuring device according to the present invention.
Fig. 2 is a perspective view of a sensory threshold measuring device.
Fig. 3 is a transverse plan view of the sensory threshold measuring device.
Fig. 4 is a longitudinal section view of a probe and a probe fixing portion.
Fig. 5 is a flowchart of primary and secondary stimulus applying conditions showing a sensory threshold measuring procedure.
Fig. 6 is a flowchart of tertiary and quaternary stimulus applying conditions showing a sensory threshold measuring procedure.
Fig. 7 is a plan view showing a measurement example of a sensory threshold.
Fig. 8 is a view illustrating measurement target positions of a sole.

### EMBODIMENTS OF THE INVENTION

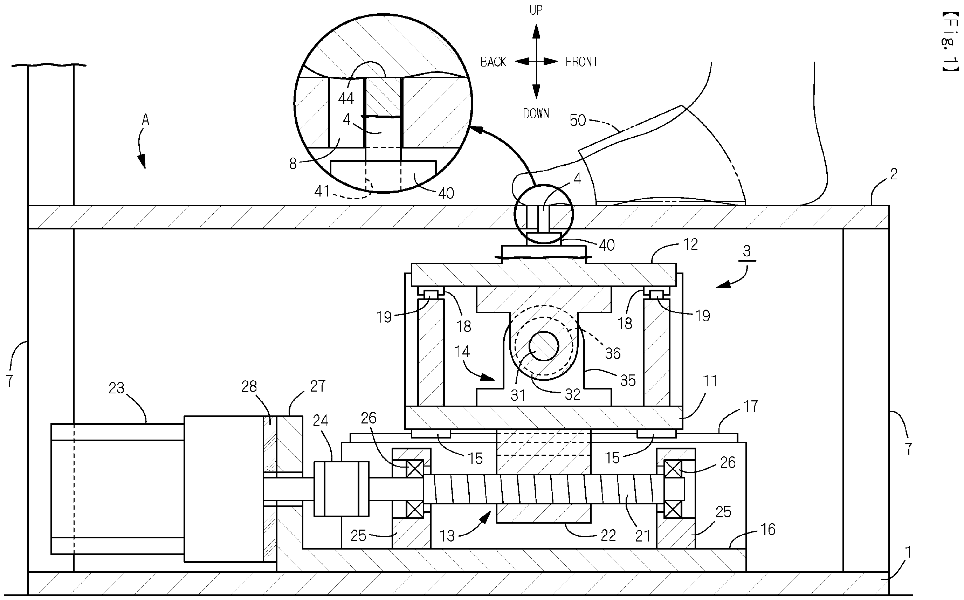

(Embodiment) Fig. 1 to Fig. 8 show an embodiment of an evaluation apparatus for diabetic peripheral neuropathy according to the present invention (hereinafter, simply referred to as "evaluation apparatus"). In the present invention, the terms front-back, left-right, and up-down are as defined by the crossing arrows, and indications of front and back, left and right, and up and down shown in drawings.

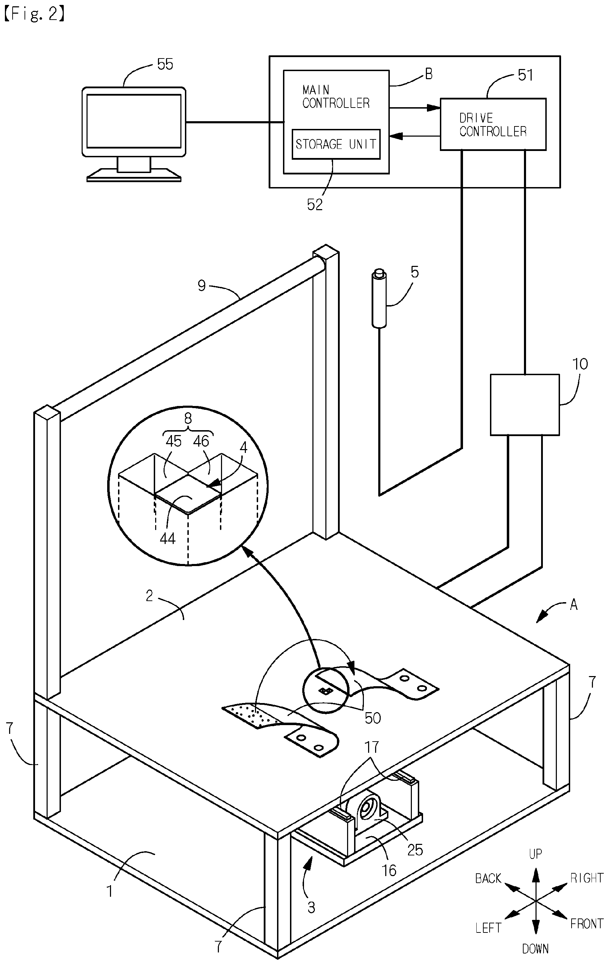

In Fig. 1 to Fig. 3, the evaluation apparatus includes a measuring device A for measuring a sensory threshold of a sole, and a main controller (computer) B for identifying a sensory threshold from a measurement result of the measuring device A and evaluating the presence or absence of neuropathy or the status of progression of neuropathy from the identified sensory threshold. The measuring device A includes a rectangular base 1, a foot pedestal 2 for supporting a sole, a probe driving structure 3 disposed between the base 1 and the foot pedestal 2, a probe 4 for applying a moving stimulus to a sole, and a input switch 5 operable by a subject. The foot pedestal 2 is formed of a thick plastic plate, and is supported fixedly by struts 7 disposed at four corners of the base 1. In the center part of the foot pedestal 2, a contact window 8 accommodating the probe 4 is open in the shape of L. On the back edge of the foot pedestal 2, a handrail 9 for supporting a subject standing on the foot pedestal 2 is disposed upright. In Fig. 2, the reference numeral 10 denotes an AD-DA converter, and the reference numeral 55 denotes a display (display means). On the top face of the foot pedestal 2, a fixing tool 50 for fixing a foot to be measured is provided. The fixing tool 50 is formed of a pair of band cloths having a hook-and-loop fastener.

The probe driving structure 3 includes a first table 11 and a second table 12 that are guided while they are reciprocally slidable in the directions that intersect at right angles along the horizontal surface, a first driving mechanism 13 for reciprocally operating the first table 11, and a second driving mechanism 14 for reciprocally operating the second table 12. The first table 11 having a rectangular shape that is long in the left-right direction is guided and supported while it is slidable in the front-back direction by a pair of left and right guide rails 17 disposed on a driving base 16 via sliders 15 provided at four positions of the bottom face of the first table 11. The second table 12 in the shape of a square is guided and supported while it is slidable in the left-right direction by a pair of front and back guide rails 19 disposed on the first table 11 via sliders 18 provided at four positions of the bottom face of the second table 12.

As shown in Fig. 1, the first driving mechanism 13 includes a ball screw shaft 21, a nut body 22 fixed on the bottom face of the first table 11, which is designed to mesh with the ball screw shaft 21, a step motor (motor) 23 for forward reverse rotary driving the ball screw shaft 21, and a coupling 24. Ends of the ball screw shaft 21 are rotatably supported by a pair of front and back bearing boxes 25 via bearings 26. The bearing boxes 25 are fixed to the driving base 16. By rotary-driving the ball screw shaft 21 forward or reverse by the step motor 23, it is possible to operate the first table 11 to move forward or backward. The step motor 23 is fixed to a bracket 27 fixed to the driving base 16 via a vibration insulating rubber (vibration insulating structure) 28, and thus, vibration occurring in the step motor 23 is blocked to prevent the vibration from being transferred to the foot pedestal 2 via the driving base 16 and the base 1, and the struts 7.

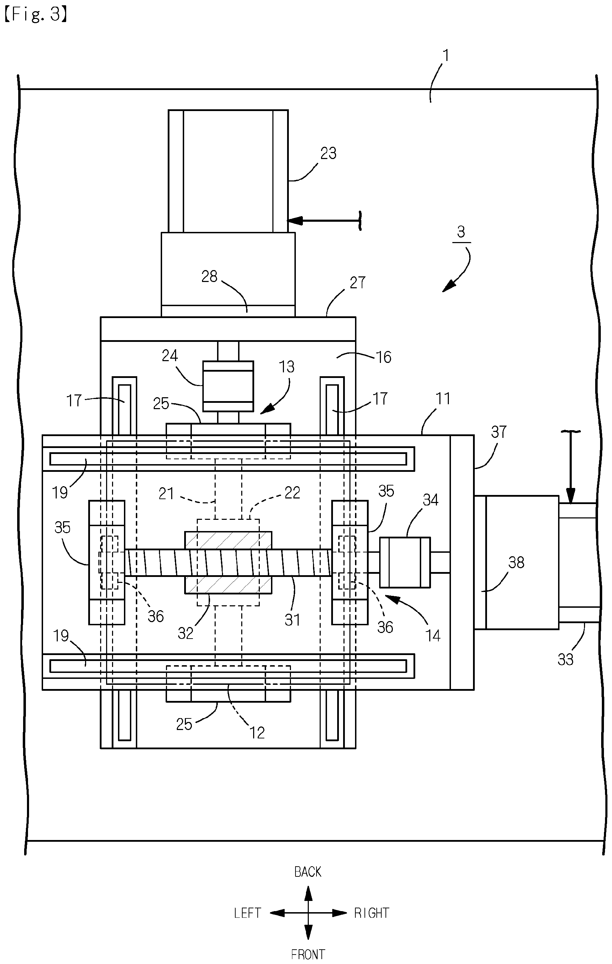

In Fig. 3, the second driving mechanism 14 includes a ball screw shaft 31, a nut body 32 fixed on the bottom face of the second table 12, which is designed to mesh with the ball screw shaft 31, a step motor (motor) 33 for forward reverse rotary driving the ball screw shaft 31, and a coupling 34. Ends of the ball screw shaft 31 are rotatably supported by a pair of left and right bearing boxes 35 via bearings 36. The bearing boxes 35 are fixed to the first table 11. By rotary-driving the ball screw shaft 31 forward or reverse by the step motor 33, it is possible to operate the second table 12 to move left or right. The step motor 33 is fixed to a bracket 37 fixed to the first table 11 via a vibration insulating rubber (vibration insulating structure) 38, and thus, vibration occurring in the step motor 33 is blocked to prevent the vibration from being transferred to the foot pedestal 2.

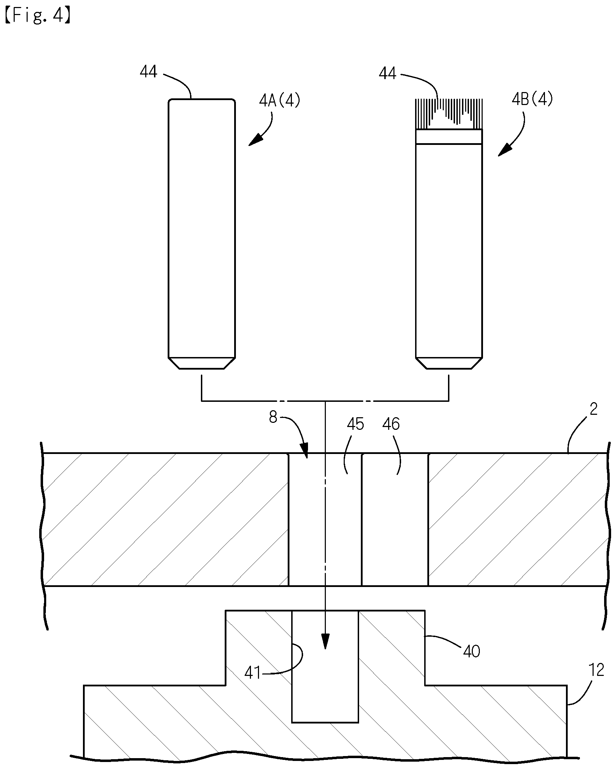

In the center of the top face of the second table 12, a probe fixing portion 40 for attaching the probe 4 is provided. As shown in Fig. 4, in the center of the probe fixing portion 40, an attachment hole 41 having a square section is formed, and by fitting the probe 4 into the attachment hole 41, the probe 4 can be moved together with front-back movement and left-right movement of the second table 12. In this embodiment, rotary power of the step motor 23 is converted into reciprocating power by the ball screw shafts 21, 31 and the nut bodies 22, 32, however, this arrangement is not necessary, and the first driving mechanism 13 and the second driving mechanism 14 may be so configured that a direct driven linear actuator such as an electric cylinder or a rodless cylinder is used as a driving source.

Fig. 4 shows two types of probes 4, and a sensory threshold can be measured by attaching these to the probe fixing portion 40. The first probe 4A (4) is made of a square-shank-shaped plastic rod, and a flat contact portion 44 provided at its upper end applies a moving stimulus to a sole. For ensuring sufficient area where the contact portion 44 contacts a sole, the front-back dimension and the left-right dimension of the contact portion 44 are set at 10 mm so that the area is 100 mm². The second probe 4B (4) is made of a square-shank-shaped plastic rod that is identical to that for the first probe 4A, and a carpet is pasted on the upper end of the rod to give a contact portion 44. The front-back dimension, the left-right dimension and the area of the contact portion 44 are as same as those of the contact portion 44 of the first probe 4A. In the state that the first probe 4A and the second probe 4B are fit into the probe fixing portion 40, the respective contact portions 44, 44 are flush with the upper opening face of the contact window 8, as shown in Fig. 1. The contact window 8 is formed into an L-shape by a front-back groove 45 and a left-right groove 46, and the front-back dimension and the left-right dimension of the grooves 45, 46 are set at 28 mm, respectively.

The input switch 5 is formed of a push button switch, which is to be turned on when the subject feels a moving stimulus during measurement of a sensory threshold by the measuring device A. An ON signal (output signal) output from the input switch 5 is taken into the main controller B, and the ON signal of the input switch 5 and the moving status of the probe 4 are stored in a storage unit 52. The ON signal of the input switch 5 and the moving status of the probe 4 are also displayed on a display (display means) 6 provided in the main controller B.

The main controller B is able to move the probe 4 separately in the front-back direction or in the left-right direction in a predetermined procedure by controlling the drive condition of the probe driving structure 3 by the drive controller 51. The moving speed of the probe 4 by the probe driving structure 3 can be set by 1 mm/s, and the moving distance can be set by 0.1 µm.

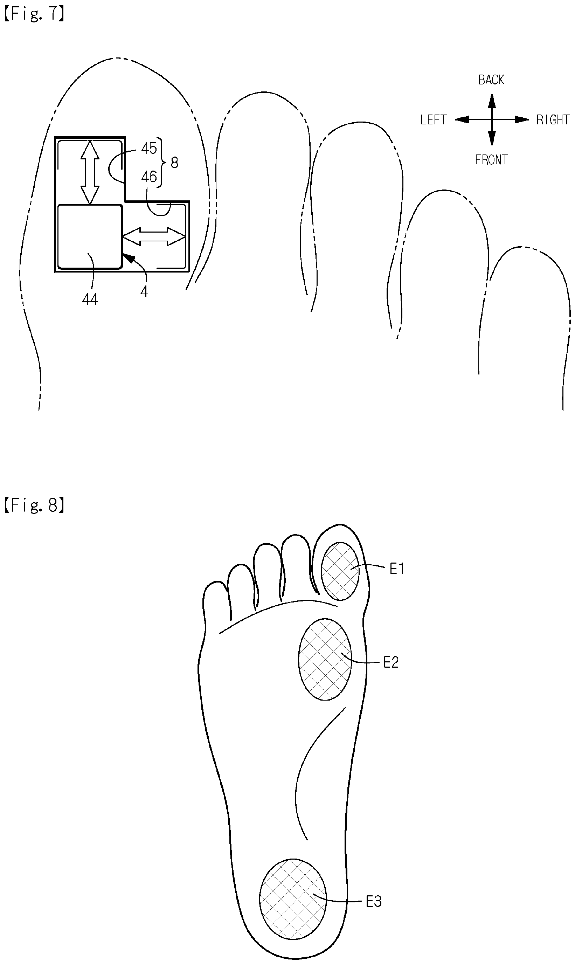

For measuring a sensory threshold in a sole by the measuring device A having the above constitution, measurement is conducted for a right sole and a left sole by using the first probe 4A. Further as shown in Fig. 7 and Fig. 8, at each position of the hallux face E1, the thenar face E2, and the heel face E3 of each foot, measurement is conducted by applying a front-back moving stimulus and a left-right moving stimulus. As the front-back moving stimulus and the left-right moving stimulus, several levels of stimuli ranging from a stimulus that is relatively difficult to be perceived, to a stimulus that is relatively easy to be perceived are prepared, and measurement of a sensory threshold is conducted while the intensity of the stimulus is gradually increased (or decreased), and further measurement is conducted while the intensity of the stimulus is randomly changed. The intensity of the moving stimulus can be changed by varying the combination of the moving speed and the moving distance of the probe 4, and which intensities of moving stimuli are applied in what order are preliminarily programmed in the main controller B.

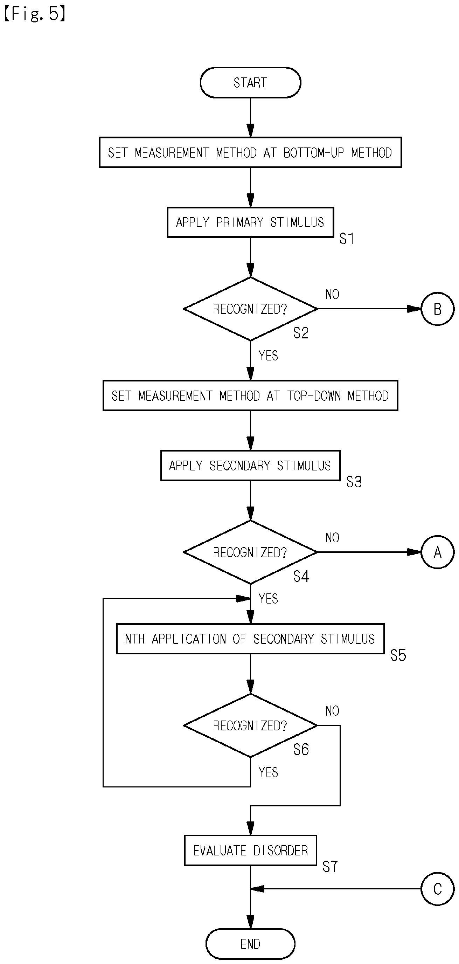

For measurement, a bare foot of a subject is placed on the foot pedestal 2 and the subject is guided to stand up in such a condition that the body weight of the subject acts on the sole. At this time, the subject grips the handrail 9 to stabilize the standing posture, while holding the input switch 5 with a hand of the dominant arm to be ready for turning on the input switch 5. Also as shown in Fig. 5, the subject is guided to stand up so that the entire hallux face E1 faces the contact window 8, and the foot to be measured is fixed by the fixing tool 50 attached to the foot pedestal 2 (see Fig. 2).

In the condition that preparation for measurement has completed, a start button of the main controller B is turned on to activate the probe driving structure 3 to thereby move the probe 4. The main controller B preliminarily stores reference data of known sensory thresholds obtained by applying a moving stimulus to a sole of patients suffering from diabetes, and age correction factors calculated from standard values of sensory thresholds based on different ages of patients, in the storage unit 52. The drive controller 51 controls drive condition of the probe driving structure 3 by using the reference data and age correction factors to sequentially conduct a primary stimulus applying condition, a secondary stimulus applying condition, and a tertiary stimulus applying condition, to thereby measure a sensory threshold.

As shown in Fig. 5, in the primary stimulus applying condition, a rough sensory threshold is temporarily set by increasing the variation range of moving stimulus by the probe 4. For example, moving stimuli of five levels are applied randomly (S1), and presence or absence of a stimulus response of the subject to the moving stimulus is checked (S2). When a stimulus response of the subject is observed (YES in S2), the smallest sensory threshold is temporarily set as a reference value. In other words, a rough sensory threshold for the moving stimuli of the five levels is temporarily set, and the condition shifts to the secondary stimulus applying condition.

In the secondary stimulus applying condition, taking the temporarily set rough sensory threshold as a reference value, a moving stimulus having a small variation range is applied to measure a sensory threshold. For example, when the temporarily set rough sensory threshold is a threshold corresponding to the level 3 among the five levels of moving stimuli, taking this as a reference value, a moving stimulus slightly smaller than the reference value is applied (S3) to check the presence or absence of a stimulus response of the subject (S4). When a stimulus response of the subject is observed (YES in S4), taking the previously applied moving stimulus as a new reference value, a moving stimulus slightly smaller than the reference value is applied (S5) to check the presence or absence of a stimulus response of the subject (S6). When a stimulus response of the subject is observed (YES in S6), a stimulus slightly smaller than the previous moving stimulus is applied (S5) to check the presence or absence of a stimulus response of the subject (S6), and then application of a smaller moving stimulus is repeated until a stimulus response of the subject is no longer observed (NO in S6) to measure a sensory threshold. The secondary stimulus applying condition can end after conducting application of a moving stimulus about five times. The obtained sensory threshold is compared and evaluated with the reference data of known sensory thresholds that are preliminarily measured in the main controller B, and the presence or absence of neuropathy in the sole, and the degree of neuropathy are determined (S7). Evaluation of the degree of neuropathy is rated, for example, on 10-point scale, and in which point of neuropathy the subject falls is identified by the main controller B, and indicated in the display 55. Concretely, the evaluated point among the 10 points of evaluation is indicated in the display 55 for an outpatient clinician, and additionally an evaluation result for the subject is displayed depending on the evaluated point, for example, "Slight neuropathy is observed." or "Strongish neuropathy is observed".

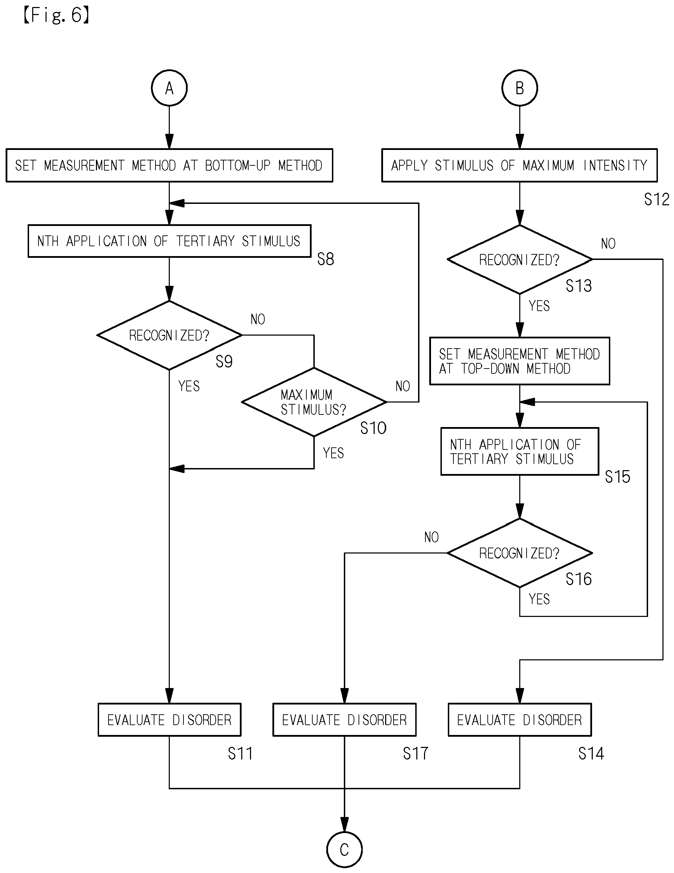

When a stimulus response of the subject is not observed (NO in S4) in the secondary stimulus applying condition, some errors or mistakes can be involved in measurement of the rough sensory threshold temporarily set in the primary stimulus applying condition. For checking this, as shown by the symbol A in Fig. 6, the condition shifts to the tertiary stimulus applying condition, and a sensory threshold is measured again by applying a moving stimulus (S8) to check the presence or absence of a stimulus response of the subject (S9). In this case, a moving stimulus larger than the moving stimulus corresponding to the sensory threshold that is temporarily set in the primary stimulus applying condition is applied, and when a stimulus response of the subject is observed (YES in S9), this is taken as a sensory threshold. The obtained sensory threshold is compared and evaluated with the reference data of known sensory thresholds that are preliminarily measured in the main controller B, and the presence or absence of neuropathy in the sole, and the degree of neuropathy are determined (S11). When a stimulus response of the subject is not observed in the tertiary stimulus applying condition (NO in S9), a stimulus larger than the previous moving stimulus is applied (S8), and the presence or absence of a stimulus response of the subject is repeatedly checked until the moving stimulus reaches the maximum value (S9). When the moving stimulus reaches the maximum value (YES in S10), the presence or absence of a stimulus response of the subject is checked at this time, and the measurement ends. Also when the moving stimulus is maximized, lack a stimulus response of the subject is regarded as anesthesia.

When a stimulus response of the subject is not observed in the primary stimulus applying condition (NO in S2), as shown by the symbol B in Fig. 6, the condition shifts to the quaternary stimulus applying condition, and a moving stimulus of the maximum intensity is applied by the probe 4 (S12) to check the presence or absence of a stimulus response of the subject (S13). When a stimulus response of the subject is observed in the quaternary stimulus applying condition (YES in S13), taking the maximum moving stimulus as a reference value, a moving stimulus smaller than the maximum moving stimulus is applied (S15) to repeatedly check the presence or absence of a stimulus response of the subject (S16), and the point when a stimulus response of the subject is no longer observed (NO in S16) is regarded as a sensory threshold. The obtained sensory threshold is compared and evaluated with the reference data of known sensory thresholds that are preliminarily measured in the main controller B, and the presence or absence of neuropathy in the sole, and the degree of neuropathy are determined (S17). Also when a stimulus response of the subject is not observed despite application of the maximum moving stimulus in the quaternary stimulus applying condition (NO in S13), it is regarded as anesthesia, and the measurement ends (S14).

As described above, for measurement of a sensory threshold, the primary stimulus applying condition, the secondary stimulus applying condition, and the tertiary stimulus applying condition are sequentially conducted, and in each stimulus applying condition, the probe 4 is reciprocated once in the front-back direction to apply a front-back moving stimulus which is relatively difficult to be perceived. When there is a significant difference in timing as a result of comparison between the timing of the output signal of the input switch 5 and the moving status of the probe 4, the output signal of the input switch 5 may be marked or invalidated as it were made by a subject's misunderstanding or an operational error.

By applying a left-right moving stimulus to the hallux face E1 in the same manner as described above, it is possible to identify a sensory threshold with respect to the left-right moving stimulus by the main controller B. After completion of measurement of the hallux face E1, the thenar face E2 is caused to face the contact window 8 as shown in Fig. 7, and measurement is conducted in the same manner as described above while a front-back moving stimulus and a left-right moving stimulus are separately applied. Further, after completion of measurement of the thenar face E2, the heel face E3 is caused to face the contact window 8, and measurement is conducted in the same manner as described above while a front-back moving stimulus and a left-right moving stimulus are separately applied, and measurement of the right foot is ended. Also for the left foot, by conducting measurement in the same manner as for the right foot while applying a front-back moving stimulus and a left-right moving stimulus to each part of the hallux face E1, the thenar face E2, and the heel face E3, it is possible to identify a sensory threshold of each measurement part.

After completion of measurement using the first probe 4A, the second probe 4B is attached to the probe fixing portion 40 in place of the first probe 4A, and sensory thresholds can be measured for the hallux face E1, the thenar face E2, and the heel face E3 of the left and right soles in the same manner as for the first probe 4A. By comparing the measurement result obtained as described above, with known sensory thresholds that are preliminarily collected and put onto a database, it is possible to determine the presence or absence of neuropathy originating in diabetes in a sole, and the degree of neuropathy. In an outpatient examination in which a time-consuming examination is difficult to be carried out, the presence or absence of neuropathy in a sole and the degree of neuropathy may be evaluated by measuring a sensory threshold only with the first probe 4A.

By preparing plural kinds of probes 4A, 4B having different physical properties of the contact portion 44, and measuring a sensory threshold while using the plural kinds of probes 4A, 4B alternatively and applying plural kinds of moving stimuli having different qualities in the stimulating and measuring step, it is possible to apply a moving stimulus appropriately depending on the situation of the sole of the subject in comparison with the case of measuring a sensory threshold for a moving stimulus only with one kind of probe 4. For example, when keratin of the sole skin is extremely thickened as is in elderly, a measurement result irrespective of the condition of the skin can be obtained by using a probe having high a coefficient of friction.

As described above, according to the measuring device having the above constitution, only by urging a subject to stand at a correct position, and turning on the start button of the main controller B, the probe 4 is operated to move by the probe driving structure 3 under the instruction from the drive controller 51 and a sensory threshold of a sole can be measured automatically and appropriately. Therefore, a sensory threshold can be measured easily by a measurer who does not have specialized knowledge of medicine or specialized knowledge and technique of bio-medical instrumentation, and additionally, variation in measurement result by different measures can be eliminated. Since anyone can appropriately measure a sensory threshold as described above, a subject can measure a sensory threshold under the guidance of a nurse or an assistant nurse while he/she is waiting for an examination in a waiting room. Therefore, it is possible to achieve an evaluation apparatus for diabetic peripheral neuropathy suited for an outpatient examination in which a time-consuming examination is difficult to be conducted. Further, since a moving stimulus set for the subject can be applied accurately by moving the probe 4 by the probe driving structure 3, it is possible to measure a sensory threshold with high reproducibility by eliminating any variation in moving stimulus.

Next, details of the evaluation method for diabetic peripheral neuropathy (hereinafter, simply referred to as "evaluation method") will be described. Measurement of a sensory threshold in a sole includes a test preparing step, a stimulating and measuring step, and an evaluating step. In the test preparing step, a sole is placed on the contact window 8 that is open at a predetermined position of the foot pedestal 2, and fixed by the fixing tool 50. In the stimulating and measuring step, the probe 4 is operated to move by the probe driving structure 3 that operates according to the control procedure of the drive controller 51, and the subject operates the input switch 5 when he/she feels a moving stimulus in the sole to measure a sensory threshold. In the evaluating step, the measured sensory threshold is evaluated in the main controller B to determine the presence or absence of neuropathy in the sole, and the degree of neuropathy.

The main controller B preliminarily stores reference data of known sensory thresholds obtained by applying a moving stimulus to a sole of patients, and age correction factors calculated from standard values of sensory thresholds based on different ages of patients. The drive controller 51 in the stimulating and measuring step controls drive condition of the probe driving structure 3 by using the previous reference data and the age correction factor to execute a primary stimulus applying condition for temporarily setting a rough sensory threshold by making the variation range of moving stimulus by the probe 4 large. Also it executes a secondary stimulus applying condition for measuring a sensory threshold by applying a moving stimulus having a small variation range while taking the rough sensory threshold obtained in the primary stimulus applying condition as a reference value. Further, when a stimulus response of the subject is not observed in the secondary stimulus applying condition, a tertiary stimulus applying condition for measuring a sensory threshold by applying a moving stimulus that is larger than the moving stimulus corresponding to the temporarily set sensory threshold in the primary stimulus applying condition is executed. In the evaluating step, the main controller B compares and evaluates the measured value obtained in the tertiary stimulus applying condition and known sensory thresholds to automatically determine the presence or absence of neuropathy in the sole, and the degree of neuropathy. Details of the measurement method in each stimulus applying condition are as previously described.

As described above, in the evaluation method, since a sensory threshold with respect to a moving stimulus is measured by appropriately using the method of limits in a stepwise manner in each stimulus applying condition, a less number of times of stimulus application on the sole is required compared with an usual method of limits, so that a sensory threshold can be measured in a shorter time. On the other hand, the less number of times of stimulus application implies the possibility that a measurement error due to habituation or expectation which is characteristic of the method of limits appears largely compared with the case of conducting measurement by the method of limits. However, in the present invention, a tertiary stimulus applying condition is executed to eliminate such a measurement error and ensure the validity of the measured value, and more accurate measurement of a sensory threshold is ensured.

More preferably, in the stimulating and measuring step, when a stimulus response of the subject is not observed in the primary stimulus applying condition, the condition shifts to the quaternary stimulus applying condition, and a moving stimulus of the maximum intensity is applied to measure a sensory threshold. When a stimulus response of the subject is observed in the quaternary stimulus applying condition, the tertiary stimulus applying condition that measures a sensory threshold by applying a moving stimulus having a small variation range while taking the maximum moving stimulus as a reference value is conducted. Thereafter, in the evaluating step, the main controller B compares and evaluates the measured known sensory thresholds and the reference data to automatically determine the presence or absence of neuropathy in the sole, and the degree of neuropathy. When a stimulus response of the subject is not observed in the quaternary stimulus applying condition, it is regarded as anesthesia, and the measurement ends.

In the stimulating and measuring step, the probe 4 is moved separately in the front-back direction and in the left-right direction by the probe driving structure 3 to apply a front-back moving stimulus and a left-right moving stimulus to a sole, and a moving distance and a moving speed of the probe 4 are measured as independent variables. According to this evaluation method, it is possible to measure a sensory threshold for a moving stimulus in the shearing direction applied to the sole in contrast to the case of a conventional measuring device that applies an oscillation stimulus or a compression stimulus on a specific site of skin. Further, it is possible to obtain a measurement result reflecting the finding that the perception characteristics of a sole differ between the two directions, and also it is possible to measure a moving distance and a moving speed of the probe 4 as independent variables. Therefore, by comparing the obtained measurement result with known sensory thresholds that are preliminarily collected and put onto a database, it is possible to identify the presence or absence of neuropathy originating in diabetes, or the status of progression of neuropathy more accurately.

In the stimulating and measuring step, it is preferred to measure a sensory threshold by separately applying a moving stimulus to each of the hallux face E1, the thenar face E2, and the heel face E3 in the sole. A moving stimulus is separately applied to each of the hallux face E1, the thenar face E2, and the heel face E3 because these three points on a sole face are the parts where sensory receptors in the skin that sense mechanical moving stimuli are densely distributed, and thresholds with respect to sensory stimulus are different from each other, and the finding that these three points have very important meanings is widely recognized. However, the present apparatus can measure any other points on a sole face than these three points in principle, and when the present apparatus is applied to diagnosis of neuropathy, for example, measurement may be conducted on a sole face other than the aforementioned three points where the peripheral nerve which is expected to be affected is dominant.

In the stimulating and measuring step, a subject is guided to stand on the foot pedestal 2, and a moving stimulus is applied in the condition that the body weight of the subject acts on the sole to measure a sensory threshold. In this manner, by applying a moving stimulus in the condition that the body weight of the subject acts on the sole, the pressure by the body weight is evenly applied to the sensory receptors distributed in the skin, and a sensory threshold is measured in a constantly stimulated condition. Since pressure application by the body weight has the effect of masking the sensitivity of sensory receptors in the skin, the sensation becomes dull, and a larger value than the sensory threshold measured in a measuring condition without application of body weight is measured. The sensory threshold measured in such a condition is exactly the value in the same condition as normal standing posture, and is effective for evaluating the presence or absence of neuropathy originating in diabetes or the status of progression of neuropathy. Such measurement is impossible with any conventional sensory function measuring techniques and measuring devices.

In the above embodiment, the probe driving structure 3 is made up of the first table 11 and the second table 12, the first driving mechanism 13 and the second driving mechanism 14 for reciprocally operating these tables 11, 12 and so on, however, this constitution is not necessary, and the probe driving structure 3 may be made up of the first table (moving table) 11, the first driving mechanism (driving structure) 13 disposed on the base 1, for reciprocally operating the first table 11, and the probe fixing portion 40 disposed on the first table 11. In this case, when the driving direction of the first table 11 is the front-back direction, for example, after applying a front-back moving stimulus to the sole of the subject with the probe 4, the posture of the subject is changed to be perpendicular to the moving direction of the probe 4, and then a left-right moving stimulus can be applied to the sole.

As described above, according to the probe driving structure 3 in which only one moving table 11 is driven, it is possible to significantly simplify the general structure of the evaluation apparatus and to achieve compactification, so that an evaluation apparatus suited for use in a narrow space such as a consultation room or a waiting room can be provided. Simplification of the structure of the evaluation apparatus is also advantageous in that the total cost can be reduced, and thus the cost for installation of the evaluation apparatus can be reduced.

Measurement of a sensory threshold in a sole is preferably conducted in the condition that the subject stands on the foot pedestal 2 so that the body weight of the subject acts on the sole, however, this is not necessary. For example, measurement of a sensory threshold may be conducted in the condition that a foot of the subject who is sitting on a chair is placed on the foot pedestal 2. In this case, it is desired to retain the foot with a belt or a holding frame provided on the foot pedestal 2 to prevent the foot from moving. The input switch 5 is not necessary a push-button switch, but other switches such as a touch switch or a lodging switch may be used. The input switch 5 may be incorporated into the handrail 9.

In the above embodiment, the case of forming the contact portion 44 of a smooth upper end face of a plastic rod, and the case of forming the contact portion 44 by pasting a carpet on the upper end of the rod are exemplified, however, the contact portion 44 is not limited to the contact portions 44 described in the embodiment. For example, a wood piece, a metal piece, surface of a tatami mat, leather, cloth, tile or the like may be pasted on the upper end of the rod to form the contact portion 44. Alternatively, the probe 4 is formed into a rod of plastic, metal or wood, and bumps and dips, grooves, projections or the like may be formed on the upper end face thereof, or the upper end face may be roughened to give the contact portion 44. In short, a large number of probes 4 which are different in physical properties such as shape, structure, coefficient of friction, hardness and the like of the contact portion 44 are prepared to apply different qualities of moving stimuli on the sole by using the probe 4 suited for the purpose of the measurement.

The motors 23, 33 of the first driving mechanism 13 and the second driving mechanism 14 are not necessarily step motors, but may be synchronous motors of other form. The vibration insulating rubbers 28, 38 can also be disposed between the driving base 16 and the base 1.

### REFERENCE SIGN LIST

- 1: Base
- 2: Foot pedestal
- 3: Probe driving structure
- 4: Probe
- 5: Input switch
- 8: Contact window
- 11: First table
- 12: Second table
- 13: First driving structure
- 14: Second driving structure
- 40: Probe fixing portion
- 44: Contact portion
- 51: Drive controller
- 52: Storage unit
- A: Measuring device
- B: Main controller (computer)

## Claims

1. An evaluation apparatus for diabetic peripheral neuropathy comprising:
a measuring device (A) for measuring a sensory threshold of a sole; and
a main controller (B) for identifying a sensory threshold from a measurement result of the measuring device (A) and evaluating presence or absence of neuropathy or status of progression of neuropathy from the identified sensory threshold, wherein
the measuring device (A) includes a foot pedestal (2) disposed on a base (1), for supporting a sole of a subject who is sitting or standing, a probe (4) for applying a moving stimulus to the sole, a probe driving structure (3) for operating movement of the probe (4), an input switch (5) to be operated by the subject recognizing the moving stimulus, and a drive controller (51) for controlling drive condition of the probe driving structure (3),
the main controller (B) preliminarily stores reference data of known sensory thresholds obtained by applying a moving stimulus to a sole of patients, and age correction factors calculated from standard values of sensory thresholds based on different ages of patients,
the drive controller (51) controls drive condition of the probe driving structure (3) by using the reference data and the age correction factors to sequentially conduct a primary stimulus applying condition, a secondary stimulus applying condition, and a tertiary stimulus applying condition to measure a sensory threshold,
in the primary stimulus applying condition, a variation range of moving stimulus by the probe (4) is set large and a rough sensory threshold is temporarily set,
in the secondary stimulus applying condition, taking the temporarily set rough sensory threshold as a reference value, a moving stimulus having a small variation range is applied to measure a sensory threshold,
in the tertiary stimulus applying condition, when a stimulus response of the subject is not observed in the secondary stimulus applying condition, a moving stimulus larger than the moving stimulus corresponding to the sensory threshold temporarily set in the primary stimulus applying condition is applied to measure a sensory threshold,
the main controller (B) compares and evaluates the measured sensory threshold and the known reference data to automatically determine the presence or absence of neuropathy in the sole, and the degree of neuropathy; wherein
the probe driving structure (3) includes a first table (11) and a second table (12) that are guided and supported while they are reciprocally slidable in a front-back direction and in a left-right direction, a first driving mechanism (13) disposed on the base (1), for reciprocally operating the first table (11), a second driving mechanism (14) disposed on the first table (11), for reciprocally operating the second table (12), and a probe fixing portion (40) disposed on the second table (12),
the probe (4) attached to the probe fixing portion (40) is separately moved in a moving direction of the first table (11) and in a moving direction of the second table (12) to measure a moving distance and a moving speed of the probe (4) as independent variables, and
the measured sensory threshold and the reference data are evaluated by the main controller (B).

2. The evaluation apparatus for diabetic peripheral neuropathy according to claim 1, further comprising: displaying means (55) that displays a determination result of the main controller (B) regarding the presence or absence of neuropathy in a sole, and the degree of neuropathy.

3. The evaluation apparatus for diabetic peripheral neuropathy according to claim 1 or 2, wherein a motor (23) constituting the first driving mechanism (13) and a motor (33) constituting the second driving mechanism (14) are respectively fixed to brackets (27, 37) via vibration insulating structures (28, 38) that block vibration, and
rotary power of the motors (23, 33) is converted into reciprocating operation by means of ball screw shafts (21, 31), and male screw bodies (22, 32) fixed to the first table (11) and the second table (12) to reciprocally slide the first table (11) and the second table (12) in the front-back direction and the left-right direction.

## Patentansprüche

1. Beurteilungsvorrichtung für diabetische periphere Neuropathie, umfassend:
eine Messvorrichtung (A) zum Messen eines sensorischen Schwellenwerts einer Sohle; und
eine Hauptsteuerung (B) zum Bestimmen eines sensorischen Schwellenwerts aus einem Messergebnis der Messvorrichtung (A) und Beurteilen des Vorhandenseins oder Nichtvorhandenseins von Neuropathie oder einem Status des Fortschreitens von Neuropathie aus dem bestimmten sensorischen Schwellenwert, wobei
die Messvorrichtung (A) ein Fußpodest (2), das auf einem Sockel (1) angeordnet ist, zum Stützen einer Sohle eines Subjekts, das sitzt oder steht, eine Sonde (4) zum Anwenden eines Bewegungsreizes für die Sohle, eine Sondenantriebsstruktur (3) zum Betreiben der Bewegung des Sonde (4), einen Eingabeschalter (5), der durch das Subjekt, das den Bewegungsreiz erkennt, zu betätigen ist, und eine Antriebssteuerung (51) zum Steuern eines Antriebszustands der Sondenantriebsstruktur (3) beinhaltet,
die Hauptsteuerung (B) vorläufig Referenzdaten von bekannten sensorischen Grenzwerten, die durch Anwenden eines Bewegungsreizes auf eine Sohle von Patienten erlangt werden, und Alterskorrekturfaktoren, die aus Standardwerten von sensorischen Schwellenwerten basierend auf Patienten unterschiedlichen Alters berechnet werden, speichert,
die Antriebssteuerung (51) einen Antriebszustand der Sondenantriebsstruktur (3) durch Verwenden der Referenzdaten und der Alterskorrekturfaktoren steuert, um nacheinander einen primären Reizanwendungszustand, einen sekundären Reizanwendungszustand und einen tertiären Reizanwendungszustand vorzunehmen, um einen sensorischen Schwellenwert zu messen,
in dem primären Reizanwendungszustand ein Variationsbereich von Bewegungsreiz durch die Sonde (4) groß eingestellt ist und ein grober sensorischer Schwellenwert vorübergehend eingestellt ist,
in dem sekundären Reizanwendungszustand, wobei der vorübergehend eingestellte grobe sensorische Schwellenwert als ein Referenzwert genutzt wird, ein Bewegungsreiz mit einem kleinen Variationsbereich angewendet wird, um einen sensorischen Schwellenwert zu messen,
in dem tertiären Reizanwendungszustand, wenn eine Reizreaktion des Subjekts bei dem zweiten Reizanwendungszustand nicht beobachtet wird, ein Bewegungsreiz größer als der Bewegungsreiz entsprechend dem sensorischen Schwellenwert, der vorübergehend in dem primären Reizanwendungszustand eingestellt ist, angewendet wird, um einen sensorischen Schwellenwert zu messen,
die Hauptsteuerung (B) den gemessenen sensorischen Schwellenwert und die bekannten Referenzdaten vergleicht und beurteilt, um automatisch das Vorhandensein oder Nichtvorhandensein von Neuropathie in der Sohle und den Grad von Neuropathie zu ermitteln; wobei
die Sondenantriebsstruktur (3) einen ersten Tisch (11) und einen zweiten Tisch (12), die geführt und gestützt werden, während sie wechselseitig in einer Vor-Zurück-Richtung und in einer Links-Rechts-Richtung verschiebbar sind, einen ersten Antriebsmechanismus (13), der am Sockel (1) zum wechselseitigen Betreiben des ersten Tischs (11) angeordnet ist, einen zweiten Antriebsmechanismus (14) der am ersten Tisch (11) zum Wechselseitigen Betreiben des zweiten Tischs (12) angeordnet ist, und einen Sondenfixierabschnitt (40), der an dem zweiten Tisch (12) angeordnet ist, beinhaltet,
die Sonde (4), die an dem Sondenfixierabschnitt (40) befestigt ist, separat in einer Bewegungsrichtung des ersten Tischs (11) und in einer Bewegungsrichtung des zweiten Tischs (12) bewegt wird, um eine Bewegungsabstand und eine Bewegungsgeschwindigkeit der Sonde (4) als unabhängige Variablen zu messen, und
der gemessene sensorische Schwellenwert und die Referenzdaten durch die Hauptsteuerung (B) beurteilt werden.

2. Beurteilungsvorrichtung für diabetische periphere Neuropathie nach Anspruch 1, ferner umfassend:
ein Anzeigemittel (55), das ein Bestimmungsergebnis der Hauptsteuerung (B) in Bezug auf das Vorhandensein oder Nichtvorhandensein von Neuropathie in einer Sohle und den Grad von Neuropathie anzeigt.

3. Beurteilungsvorrichtung für diabetische periphere Neuropathie nach Anspruch 1 oder 2, wobei ein Motor (23), der den ersten Antriebsmechanismus (13) darstellt, und ein Motor (33), der den zweiten Antriebsmechanismus (14) darstellt, jeweils über vibrationsisolierte Strukturen (28, 38), die Vibrationen blockieren, an Klammern (27, 37) befestigt sind, und
Drehleistung der Motoren (23, 33) in wechselseitigen Betrieb durch Kugelumlaufspindeln (21, 31) und Außengewindeschraubenkörper (22, 32), die an dem ersten Tisch (11) und dem zweiten Tisch (12) befestigt sind, umgewandelt wird, um den ersten Tisch (11) und den zweiten Tisch (12) wechselseitig in der Vor-Zurück-Richtung und der Links-Rechts-Richtung zu verschieben.

## Revendications

1. Appareil d'évaluation pour la neuropathie périphérique diabétique comprenant :
un dispositif de mesure (A) pour mesurer un seuil sensoriel d'une plante ; et
un dispositif de commande principal (B) pour identifier un seuil sensoriel à partir d'un résultat de mesure du dispositif de mesure (A) et évaluer la présence ou l'absence de neuropathie ou l'état de progression de la neuropathie à partir du seuil sensoriel identifié,
ledit dispositif de mesure (A) comprenant un support de pied (2) disposé sur une base (1) pour supporter la plante d'un sujet qui est assis ou debout, une sonde (4) pour appliquer un stimulus de déplacement à la plante, une structure d'entraînement de sonde (3) pour actionner le déplacement de la sonde (4), un commutateur d'entrée (5) à être actionné par le sujet reconnaissant le stimulus de déplacement et un dispositif de commande d'entraînement (51) pour commander une condition d'entraînement de la structure d'entraînement de sonde (3),
ledit dispositif de commande principal (B) stockant préalablement des données de référence des seuils sensoriels connus obtenues en appliquant un stimulus de déplacement à une plante de patients et des facteurs de correction liés à l'âge calculés à partir de valeurs standards de seuils sensoriels sur la base des différents âges des patients,
ledit dispositif de commande d'entraînement (51) commandant la condition d'entraînement de la structure d'entraînement de sonde (3) en utilisant les données de référence et les facteurs de correction liés à l'âge pour piloter séquentiellement une condition d'application de stimulus primaire, une condition d'application de stimulus secondaire et une condition d'application de stimulus tertiaire pour mesurer un seuil sensoriel,
dans la condition d'application de stimulus primaire, une plage de variation de stimulus de déplacement par la sonde (4) étant définie pour être large et un seuil sensoriel approximatif étant défini temporairement,
dans la condition d'application de stimulus secondaire, considérant le seuil sensoriel approximatif défini temporairement en tant que valeur de référence, un stimulus de déplacement possédant une petite plage de variation étant appliqué pour mesurer un seuil sensoriel,
dans la condition d'application de stimulus tertiaire, lorsqu'une réponse du sujet à un stimulus n'est pas observée dans la condition d'application de stimulus secondaire, un stimulus de déplacement, plus grand que le stimulus de déplacement correspondant au seuil sensoriel temporairement défini dans la condition d'application de stimulus primaire étant appliqué pour mesurer un seuil sensoriel,
ledit dispositif de commande principal (B) comparant et évaluant le seuil sensoriel mesuré et les données de référence connues pour déterminer automatiquement la présence ou l'absence de neuropathie dans la plante et le degré de neuropathie ;
ladite structure d'entraînement de sonde (3) comprenant une première table (11) et une seconde table (12) qui sont guidées et supportées tout en pouvant coulisser en va-et-vient selon un direction avant-arrière et selon une direction gauche-droite, un premier mécanisme d'entraînement (13) disposé sur la base (1), pour actionner en va-et-vient la première table (11), un second mécanisme d'entraînement (14) disposé sur la première table (11) pour actionner en va-et-vient la seconde table (12) et une partie de fixation de sonde (40) disposée sur la seconde table (12),
ladite sonde (4) fixée à la partie de fixation de sonde (40) étant déplacée séparément selon une direction de déplacement de la première table (11) et selon une direction de déplacement de la seconde table (12) pour mesurer une distance de déplacement et une vitesse de déplacement de la sonde (4) sous forme de variables indépendantes, et
ledit seuil sensoriel mesuré et lesdites données de référence étant évalués par le dispositif de commande principal (B).

2. Appareil d'évaluation pour la neuropathie diabétique périphérique selon la revendication 1, comprenant en outre :
un moyen d'affichage (55) qui affiche un résultat de détermination du dispositif de commande principal (B) concernant la présence ou l'absence de neuropathie dans une plante et le degré de neuropathie.

3. Appareil d'évaluation pour la neuropathie diabétique périphérique selon la revendication 1 ou 2, un moteur (23) constituant le premier mécanisme d'entraînement (13) et un moteur (33) constituant le second mécanisme d'entraînement (14) étant respectivement fixés aux supports (27, 37) par l'intermédiaire de structures d'isolation contre les vibrations (28, 38) qui bloquent les vibrations, et
ladite puissance de rotation des moteurs (23, 33) étant convertie en un actionnement en va-et-vient au moyen d'arbres de vis à billes (21, 31) et de corps de vis mâles (22, 32) fixés à la première table (11) et à la seconde table (12) pour faire coulisser en va-et-vient la première table (11) et la seconde table (12) selon la direction avant-arrière et la direction gauche-droite.
